(19) Europäisches Patentamt
European Patent Office
Office européen des brevets

(11) EP 0 628 531 B1

(12) FASCICULE DE BREVET EUROPEEN

(45) Date de publication et mention de la délivrance du brevet: **29.10.1997 Bulletin 1997/44**

(51) Int. Cl.[6]: **C07C 43/04**, C07C 43/11, C07C 41/16

(21) Numéro de dépôt: **94401189.9**

(22) Date de dépôt: **30.05.1994**

# (54) Procédé d'éthérification sélective d'un mono ou d'un polyalcool primaire ou secondaire

Verfahren zur selektiven Etherifizierung eines primären oder sekundären Mono- oder Polyalkohols

Process for the selective etherification of a primary of secondary mono- or polyalcohol

(84) Etats contractants désignés:
**AT BE CH DE DK ES GB GR IE IT LI LU MC NL PT SE**

(30) Priorité: **09.06.1993 FR 9306929**

(43) Date de publication de la demande:
**14.12.1994 Bulletin 1994/50**

(73) Titulaire: **PRODUITS CHIMIQUES DU SIDOBRE-SINNOVA**
**Saint Fargeau Ponthierry (Seine et Marne) (FR)**

(72) Inventeurs:
- **Abribat, Benoît**
**Mons (Haute Garonne) (FR)**
- **Le Bigot, Yves**
**Toulouse (Haute Garonne) (FR)**
- **Gaset, Antoine**
**Toulouse (Haute Garonne) (FR)**
- **Olivera, François Xavier**
**Melun (Seine et Marne) (FR)**

(74) Mandataire: **Cabinet HERRBURGER**
**115, Boulevard Haussmann**
**75008 Paris (FR)**

(56) Documents cités:
**EP-A- 0 021 497**
**EP-A- 0 300 527**
**DE-A- 3 606 173**
**DE-C- 4 138 166**

- **SYNTHESIS, no. 2, février 1979, Stuttgart, DE, pages 123 - 124, B. G. ZUPANCIC et al: "Zur Glykol-Veretherung im Fest-Flüssig-Zweiphasensystem"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Rank Xerox (UK) Business Services
2.14.18/3.4

## Description

La présente invention concerne un procédé d'éthérification sélective d'un mono ou d'un polyalcool primaire ou secondaire ROH quelconque polyoxygéné ou non de façon à le transformer en un éther correspondant ROR'.

Les alcools ou, de façon plus générale, les composés organiques possédant des fonctions hydroxyles trouvent de nombreuses applications dans une large gamme de branches de la chimie. Pour certaines de ces applications, parmi lesquelles on peut citer, à titre d'exemple, les industries papetières, textiles ou encore la peinture ..., il est souhaitable de bloquer les fonctions alcools de ces composés hydroxylés : l'existence de groupes OH libres peut, en effet, s'avérer gênante en particulier en induisant des réactions secondaires ; il serait donc intéressant de pouvoir modifier la structure de certains produits pour changer leur comportement physico-chimique en les rendant par exemple plus hydrophobes.

Les réactions d'éthérification des alcools sont des réactions classiques dans le domaine de la synthèse organique, et les spécialistes connaissent, en particulier depuis longtemps les réactions de type Williamson par lesquelles dans un premier temps, on transforme, en présence d'une base forte, l'alcool ROH en alcoolate RO- et dans un second temps, on transforme l'alcoolate RO- en un éther ROR' par substitution à l'aide d'un dérivé halogéné R'X.

De façon traditionnelle, ces réactions sont mises en oeuvre en milieu liquide homogène et en présence de solvants organiques ; pour obtenir des rendements suffisamment élevés, leur mise en oeuvre exige des conditions opératoires très sévères et, en particulier, l'utilisation de bases très fortes comme les hydrures ou les alcoolates métalliques et d'halogénures très réactifs tels que par exemple le chlorure de méthyle.

Ces conditions font que ces réactions sont difficilement transposables à l'échelle industrielle. En conséquence, la mise en oeuvre de ces réactions était, jusqu'à présent, essentiellement envisagée pour des produits de grande valeur commerciale.

Les spécialistes ont déjà effectué de nombreuses tentatives dans le but d'adoucir les conditions opératoires nécessaires à la mise en oeuvre des réactions d'éthérification de type Williamson, mais aucune n'a, jusqu'à présent, permis de donner entière satisfaction, si ce n'est dans le cas de l'éthérification de structures particulièrement réactives telles qu'à titre d'exemple les composés phénoliques dont le proton est relativement facile à arracher compte tenu de sa forte acidité.

Parmi les tentatives susmentionnées, certains auteurs ont préconisé une mise en oeuvre en milieu hétérogène liquide/liquide en présence d'une phase aqueuse.

Cette mise en oeuvre qui requiert un catalyseur de transfert de phase (sels d'ammonium quaternaires, étherscouronnes, cryptands) présente de graves défauts, essentiellement liés à la présence de la phase aqueuse (corrosité du milieu, réactions secondaires, obligation d'utilisation de catalyseurs qui se dégradent, difficulté d'extraction des produits formés lorsqu'ils sont solubles en milieu aqueux, ...).

D'autres auteurs ont proposé de travailler en présence d'une base fixée sur support solide, les réactifs étant solubilisés dans un solvant organique.

Dans ce cas, les rendements sont généralement limités et les installations sont d'encombrement important rapporté aux quantités de produits obtenus. De plus, l'entité basique doit être régénérée, ce qui conduit à des effluents gênants.

Une voie plus récente de mise en oeuvre des réactions de Williamson consiste à réaliser celles-ci en milieu hétérogène triphasique solide/liquide/ liquide.

Dans ce cas, le catalyseur, généralement un polyéther, est fixé sur un support polymérique et la base est à l'état dissous dans la phase aqueuse.

L'avantage de cette mise en oeuvre, par rapport à la précédente, réside dans la facilité d'extraction du catalyseur et sa plus grande stabilité. Toutefois, le coût particulièrement élevé de ces catalyseurs greffés sur support limite leurs usages à la synthèse en laboratoire. De plus, les inconvénients provenant de la présence d'une phase aqueuse subsistent.

Par ailleurs, d'autres travaux font état de l'utilisation d'un milieu biphasique solide/liquide mais leur application reste limitée à des composés de structures bien particulières connus pour leur réactivité élevée : MeX, $Me_2SO_4$ (DE-A-36 06 173, DE-C-41 38 166), $C_6H_5CH_2X$, $H(OCH_2CH_2)_nX$ (EP-A-0 300 527) ; ces procédés ne prévoient ni taux d'hydratation particulier, ni activation par les oxygènes d'un polyéther.

L'action d'un alcoolate préformé ou commercial sur un dérivé halogéné, décrite en 1984 par BRAM et Coll, permet la préparation d'éthers en l'absence de solvant. Cette technique qui nécessite la formation préalable de l'alcoolate et la présence d'un catalyseur de transfert de phase, conduit, en outre, au développement de réactions secondaires, en particulier aux réactions d'élimination à partir du dérivé halogéné.

Il est également à noter que l'on connaît, par le document EP-A-0 300 527, un procédé de synthèse d'un monoalcool primaire particulier et plus précisément d'un mono-éther de polyoxyalkylèneglycol ayant un nombre prédéterminé d'unités d'oxyalkylène, ce en protégeant la fonction alcool terminale pendant toute la réaction de synthèse ; cependant, par ce procédé, on ne cherche pas à bloquer par éthérification les fonctions alcools de composés hydroxylés quelconques.

L'invention a pour objet de remédier à ces inconvénients, en proposant un procédé sélectif d'éthérification de type

Williamson, simple et peu onéreux, donc aisément transposable à grande échelle et parallèlement généralisable à tout composé possédant une ou plusieurs fonctions hydroxyles primaires ou secondaires, et ce quelle que soit la chaîne alkylée de l'halogénure.

Selon l'invention, ce procédé est caractérisé en ce que l'on fait réagir, en une seule étape à pression atmosphérique et sous agitation, l'entité basique sous forme solide, de préférence finement divisée, l'alcool à éthérifier et l'halogénure dans un milieu légèrement hydraté exempt de solvant et en présence d'une faible quantité d'un polyéther faisant office de catalyseur, puis, on isole l'éther formé de façon connue en elle-même, étant entendu que ni l'alcool de départ ROH ni l'éther recherche ROR' ne sont le polyéther faisant office de catalyseur.

Les quantités de catalyseur mises en oeuvre peuvent avantageusement être de l'ordre de 5 à 10 % par rapport à la masse de l'alcool à éthérifier.

La particularité du procédé conforme à l'invention est avant tout liée à l'absence de solvant et au choix d'un milieu réactionnel biphasique solide/liquide à taux d'hydratation contrôlé.

Il est en effet à noter qu'il a toujours existé un préjugé à l'encontre des réactions mises en oeuvre en l'absence de solvant qui sont, traditionnellement, considérées comme imprévisibles.

Or, conformément à l'invention, on s'est rendu compte que, de manière surprenante, l'addition d'un polyéther dans des conditions catalytiques associé à une légère hydratation du milieu réactionnel, permettait d'activer suffisamment le cation de la base pour permettre la formation exclusive de l'alcoolate et l'obtention rapide et sélective de l'éther en une seule étape de synthèse et dans un réacteur unique.

Les avantages d'un tel processus par comparaison aux processus traditionnels mis en oeuvre en présence de solvants et en phase liquide/liquide sont évidents compte tenu, en particulier, de la possibilité d'éliminer, en fin de processus, la base qui se trouve à l'état solide par simple filtration, et ainsi d'éviter de devoir mettre en oeuvre des séparations finales longues et coûteuses.

De plus, les solvants subissent, à l'heure actuelle, des interdictions de plus en plus nombreuses en milieu industriel de façon à accroître la sécurité et à diminuer les risques de pollution de l'environnement ; par suite, les processus industriels pouvant être mis en oeuvre dans des milieux exempts de solvant sont toujours très appréciés par les spécialistes.

Conformément à l'invention, les molécules d'eau présentes dans le milieu réactionnel hydratent le cation de la base solide. Ce cation hydraté est lui-même solvaté par les oxygènes du polyéther utilisé en tant que catalyseur, ce qui permet l'activation de l'anion associé de la base de façon à lui permettre d'arracher le proton de la fonction alcool à éthérifier, pour former l'alcoolate. La présence du cation solvaté permet, parallèlement, d'activer la réaction de substitution sur le dérivé halogéné R'X correspondant à la seconde phase de la réaction de Williamson et permettant d'obtenir l'éther recherché.

En d'autres termes, l'effet ionisant des molécules d'eau se conjugue à l'effet dissociant lié à la présence des atomes d'oxygène du polyéther de façon à exalter la basicité de l'anion de la base solide par solvatation du cation associé de façon à permettre l'obtention de l'éther avec des rendements élevés et des conditions opératoires d'une grande simplicité de mise en oeuvre.

On a pu constater que le procédé d'éthérification conforme à l'invention est transposable à l'éthérification de tous les alcools primaires et secondaires : les rendements sont tout naturellement plus élevés dans le cas des alcools primaires compte tenu du fait que leur proton est plus acide ; la réaction ne marche pratiquement pas avec les alcools tertiaires dans lesquels le proton est très peu acide et dans lesquels on est, de plus, gêné par des problèmes d'encombrement stérique.

Un cas particulier du procédé conforme à l'invention correspond à l'éthérification des fonctions alcool d'un polyéther hydroxylé : en effet, dans un tel cas, les fonctions éther présentes dans la structure de l'alcool à éthérifier agissent également en tant que catalyseur.

Les polyéthers hydroxylés et notamment les polyéthylèneglycols et les polypropylèneglycols sont très utilisés industriellement, en particulier dans le domaine des détergents. L'invention pourrait par suite trouver de nombreuses applications.

Conformément à l'invention, on cherche bien entendu à obtenir des rendements qui soient les plus élevés possibles, et en particulier dans ce but, à augmenter la sélectivité de la réaction de formation de l'alcoolate, c'est-à-dire à favoriser la réaction acido-basique d'arrachement du proton de l'alcool à éthérifier par l'anion de la base aux dépens de la réaction nucléophile concurrente de substitution sur l'halogénure également présent dans le milieu réactionnel.

A cet effet, il convient de fixer judicieusement les différents paramètres qui interviennent dans la mise en oeuvre de la réaction, à savoir les natures, taux et quantités mis en oeuvre des différents réactifs et catalyseurs ainsi que les conditions opératoires.

Comme il a déjà été indiqué, on a pu se rendre compte, conformément à l'invention, que la quantité de catalyseur mise en oeuvre pouvait avantageusement être de l'ordre de 5 à 10 % par rapport à la masse de l'alcool à éthérifier.

Cette condition a pu, à titre d'exemple, être vérifiée grâce à un essai par lequel on a fait réagir, pendant 4 heures et sans apport thermique, 0,126 mole, soit 20 g de 1 décanol, 0,189 mole de potasse, soit 1,5 fois la quantité stoechiométrique et 0,252 mole de bromure de butyle soit 2 fois la quantité stoechiométrique et en choisissant en tant que catalyseur du polyéthylèneglycol 350 préalablement éthérifié.

Les rendements en éther obtenus par rapport à la quantité de catalyseur mise en oeuvre ont été déterminés, après filtration, par analyse chromatographique en phase vapeur ; ils sont indiqués dans le tableau I ci-dessous :

TABLEAU I

| Quantité de catalyseur en g | % massique | Rendement % |
|---|---|---|
| 0 | 0 | 15 |
| 0,5 | 2,5 | 51 |
| 1 | 5 | 74 |
| 2 | 10 | 90 |
| 5 | 25 | 90 |
| 10 | 50 | 90 |
| 20 | 100 | 90 |

Ce tableau montre que l'addition de seulement 5 % de catalyseur par rapport à la masse d'alcool de départ, permet déjà de multiplier par 5 le rendement de la réaction.

L'étude de l'avancement de la réaction d'éthérification en fonction du temps et dans des conditions opératoires identiques à celles précisées dans l'essai précédent montre, pour sa part comme cela ressort des résultats expérimentaux rapportés sur les courbes représentées en figure 1, que la réaction évolue rapidement, essentiellement en début de réaction.

Un autre paramètre essentiel de la réaction conforme à l'invention est lié au choix du polyéther mis en oeuvre en tant que catalyseur. Il est, en effet, essentiel que celui-ci ait un nombre d'atomes d'oxygène suffisant pour solvater, c'est-à-dire "enrober" le cation de la base de façon à permettre une activation de l'anion correspondant et que cet "enrobage" ne soit pas empêché par des problèmes d'encombrement stérique liés à l'éventuelle présence de ramifications sur le squelette du polyéther.

On a ainsi pu constater, selon une autre caractéristique de l'invention, que le polyéther devait comporter au moins 5 groupements éthoxy consécutifs donc 6 atomes d'oxygène par molécule.

Ce résultat a pu être confirmé par une série d'essais pour laquelle on a fait réagir sous agitation, pendant 5 heures à 25°C, 0,063 mole de 1-décanol (158 g par mole), 0,095 mole de potasse, 0,126 mole de bromure de butyle et une quantité de 5 % d'un catalyseur par rapport à la masse d'alcool. Après filtration et analyse chromatographique en phase vapeur, on a déterminé le rendement de l'éthérification dans le cas de différents polyéthers se distinguant par le nombre d'atomes d'oxygène de leur molécule. Les résultats obtenus sont rassemblés dans le tableau II ci-dessous :

TABLEAU II

| Polyéther | Nombre moyen d'atomes d'oxygène par molecule | Rendement % |
|---|---|---|
| Sans | 0 | 14 |
| Diéthylèneglycol diméthyléther | 3 | 19 |
| Diéthylèneglycol dibutyléther | 3 | 18 |
| Triéthylèneglycol diméthyléther | 4 | 28 |
| Tétraéthylèneglycol diméthyléther | 5 | 50 |
| Polyéthylèneglycol 200 | 5 | 32 |
| Polyéthylèneglycol 300 | 7,5 | 69 |
| Polyéthylèneglycol 350 | 8,5 | 75 |
| Polyéthylèneglycol 400 | 10 | 79 |
| Polyéthylèneglycol 600 | 14 | 80 |

Ces essais montrent clairement que les rendements augmentent très rapidement avec le nombre d'oxygène par molécule du polyéther employé et confirme qu'il est nécessaire d'avoir une longueur de chaîne minimale ou, plus précisément, un nombre d'atomes d'oxygène minimum pour séparer les paires d'ions de la base et, en particulier, entourer le cation potassium.

Les résultats obtenus conformément à ces essais ont pu être confirmés grâce à des essais similaires dans lesquels l'alcool à éthérifier était le 1-dodécanol.

Conformément à ces essais, on a fait réagir sous agitation, pendant 5 heures à 25°C, 0,027 mole de 1-dodécanol (186,33 g par mole), 0,040 mole de potasse, 0,054 mole de bromure de butyle et 5 % par rapport à la masse d'alcool d'un polyéther faisant office de catalyseur.

Après filtration et analyse chromatographique en phase vapeur, on a déterminé le rendement de l'éthérification en fonction du nombre d'atomes d'oxygène de la molécule du polyéther utilisé en tant que catalyseur. Les résultats obtenus sont rassemblés dans le tableau III ci-dessous :

TABLEAU III

| Polyéther | Nombre moyen d'atomes d'oxygène par molécule | Rendement % |
|---|---|---|
| Sans | 0 | 14 |
| Triéthylèneglycol diméthyléther | 4 | 27 |
| Tétraéthylèneglycol diméthyléther | 5 | 52 |
| Polyéthylèneglycol 300 | 7,5 | 69 |
| Polyéthylèneglycol 400 | 10 | 80 |
| Polyéthylèneglycol 600 | 14 | 86 |

Ces résultats confirment entièrement ceux obtenus précédemment, à savoir que la longueur et plus particulièrement le nombre d'atomes d'oxygène du catalyseur est primordial à l'éthérification du 1-dodécanol.

Pour compléter les essais susmentionnés, on a effectué une série d'essais similaires aux deux séries d'essais précédentes mais en choisissant cette fois en tant qu'alcool à éthérifier non pas un alcool primaire, mais un alcool secondaire, le 2-octanol.

On a ainsi fait réagir, sous agitation pendant 5 heures à 25°C, 0,077 mole de 2-octanol (130,23 g par mole), 0,115 mole de potasse, 0,154 mole de bromure de butyle et une quantité de 5 % par rapport à la masse d'alcool d'un polyéther faisant office de catalyseur. Après filtration et analyse chromatographique en phase vapeur, on a déterminé le rendement de l'éthérification en fonction du nombre d'atomes d'oxygène du polyéther choisi en tant que catalyseur.

Les résultats obtenus sont rassemblés dans le tableau IV ci-dessous :

TABLEAU IV

| Polyéther | Nombre moyen d'atomes d'oxygène par molécule | Rendement % |
|---|---|---|
| Sans | 0 | 15 |
| Diéthylèneglycol diméthyléther | 3 | 15 |
| Triéthylèneglycol diméthyléther | 4 | 20 |
| Tétraéthylèneglycol diméthyléther | 5 | 21 |
| Polyéthylèneglycol 200 | 5 | 16 |
| Polyéthylèneglycol 300 | 7,5 | 34 |
| Polyéthylèneglycol 350 | 8,5 | 46 |
| Polyéthylèneglycol 400 | 10 | 46 |
| Polyéthylèneglycol 600 | 14 | 50 |
| Polyéthylèneglycol 1000 | 23 | 56 |

On peut, là encore, se rendre compte que le rendement augmente avec le nombre d'oxygène de la molécule du polyéther choisi en tant que catalyseur ; de plus, les résultats obtenus sont clairement inférieurs à ceux obtenus dans le cas des alcools primaires, ce qui est normal compte tenu de la plus faible acidité du proton de la fonction alcool.

Indépendamment de ce qui précède, il convient de se pencher sur la nature chimique du polyéther utilisé comme catalyseur, et en particulier sur le caractère ramifié ou non de sa chaîne.

On a ainsi pu constater que, à l'exception du cas particulier susmentionné qui correspond en fait à une autocatalyse, seuls peuvent être utilisés en tant que catalyseurs, les polyéthylèneglycols $H(OCH_2CH_2)_nOH$, et que les polypropylèneglycols

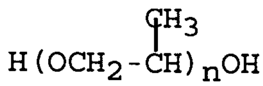

ne permettent pas d'obtenir des rendements d'éthérification intéressants ; ce mauvais résultat est, selon toute probabilité, consécutif à des problèmes d'encombrement qui empêchent d'obtenir un "enrobage" satisfaisant du cation de la base.

La situation est différente dans le cas de l'autocatalyse et le procédé permet alors d'éthérifier les groupes OH des polypropylèneglycols hydroxylés.

Cette possibilité a pu être confirmée par un essai comparatif par lequel on a fait réagir sous agitation à température ambiante et pendant 5 heures, des polyéthers hydroxylés, de la potasse et du bromure de butyle, ces deux derniers composés étant en quantité double par rapport à la stoechiométrie, et, après filtration et traitement à l'évaporateur rotatif, pour éliminer le dérivé halogéné en excès, on a déterminé le rendement par mesure des indices d'hydroxyle du composé de départ et du composé éthérifié. Les résultats obtenus sont rassemblés dans le tableau ci-dessous :

TABLEAU V

| Polyéther traité | Rendement % |
|---|---|
| Polyéthylèneglycol 400 | 100 |
| Polypropylèneglycol 725 | 80 |
| Polypropylèneglycol 1000 | 85 |
| Polypropylèneglycol Triol 725 | 76 |
| Polypropylèneglycol Triol Polyéthylèneglycol CAPPED 2800 | 96 |
| Polypropylèneglycol Triol Polyéthylèneglycol CAPPED 4800 | 77 |

Le rendement est de 95 % lors de l'éthérification de polyéthylèneglycols à l'aide de quantités stoechiométriques de réactifs. En outre, la sélectivité de cette réaction se traduit par l'obtention d'un taux de conversion de 100 % du dérivé halogéné en éther lors de l'utilisation de potasse et de bromure d'alkyle à raison de 0,6 fois la stoechiométrie par rapport à un polyoxyalkylène à fonction hydroxyle primaire.

Ces essais montrent que le blocage des fonctions hydroxyles primaires ou secondaires conformément à l'invention semble généralisable à tout type de polymère alkoxylé.

On a également pu remarquer que la réaction devient très exothermique (supérieure à 100°C) dans le cas de l'alkylation de fonctions hydroxyles primaires d'un polyéther hydroxylé comportant majoritairement des fonctions éthoxy.

Outre, le catalyseur, un autre paramètre essentiel lors de la mise en oeuvre du procédé conforme à l'invention correspond au taux d'hydratation du milieu réactionnel.

On a ainsi pu se rendre compte, conformément à une autre caractéristique de l'invention, que le taux d'hydratation du milieu réactionnel devait, de préférence, être choisi de sorte que le rapport du nombre de moles d'eau au nombre de moles de base forte soit compris entre 0,1 et 5.

Cette situation a pu être confirmée grâce à une série d'essais comparatifs dans laquelle on a fait réagir pendant 3 heures à une température de 20°C, 0,0625 mole de polypropylèneglycol 1000, soit 0,125 mole de fonction hydroxyle, 0,437 mole de potasse soit 3,5 fois la quantité stoechiométrique et 0,250 mole de bromure de butyle ; en fin de réaction, on a mesuré à partir des indices d'hydroxyle le rendement en composés éthérifiés.

Les résultats obtenus sont rassemblés dans le tableau VI ci-dessous. Les taux d'hydratation ont été déterminés à l'instant initial par la méthode de Karl Fisher :

TABLEAU VI

| Taux d'hydratation % | Rendement % |
|---|---|
| 0,9 | 74 |
| 7 | 92 |
| 15 | 95 |
| 30 | 54 |

Ce tableau montre que la présence de l'eau est importante puisqu'elle modifie le rendement optimum de 20 %, mais qu'il convient, néanmoins, de ne pas opérer en milieu trop fortement aqueux pour, d'une part, ne pas trop déplacer l'équilibre ROH/RO- en direction de l'alcool de départ et, d'autre part, ne pas risquer de dissoudre une partie de la base.

Pour confirmer ce résultat, on a effectué des essais similaires en faisant réagir, pendant 4 heures et sans apport thermique, 20 g de 1-décanol soit 0,126 mole, 0,189 mole de potasse soit 1,5 fois la quantité stoechiométrique et 0,252 mole de bromure de butyle soit 2 fois la quantité stoechiométrique, d'une part, sans addition de catalyseur et, d'autre part, en ajoutant 10 % de polyéthylèneglycol 350 préalablement éthérifié par rapport à la masse de l'alcool. On a, ensuite comme précédemment, déterminé le rendement de l'éthérification en fonction du taux d'hydratation initial du milieu réactionnel déterminé par le méthode de Karl Fischer.

Les résultats obtenus ont été exprimés par les courbes 1 et 2 représentées en figure 2.

La courbe 1 représente les variations du rendement de la réaction d'éthérification en fonction du taux d'hydratation du milieu réactionnel en l'absence de catalyseur tandis que la courbe 2 représente ces mêmes variations en présence de catalyseur.

Ces courbes montrent clairement le rôle très important de la solvatation du cation de la base. Il s'avère, en effet, que l'action du catalyseur sur le cation potassium permet de multiplier par 5 le rendement de la réaction. Le catalyseur n'agit, cependant, pas seul et son effet est conjugué à celui des molécules d'eau puisse, dans tous les cas, on observe un maximum de rendement pour un taux d'hydratation déterminé.

Conformément à l'invention, on a également eu l'idée de rechercher si la nature et la granulométrie de la base forte solide mise en oeuvre conformément à l'invention pouvaient avoir une influence sur les rendements obtenus.

On a ainsi pu constater qu'il est particulièrement avantageux d'utiliser un hydroxyde alcalin ou alcalino-terreux et, de préférence la potasse, qui permet d'obtenir des rendements nettement supérieurs à ceux obtenus avec la soude ou avec l'hydroxyde de baryum.

A titre d'exemple, des essais similaires à l'essai susmentionné relatif à l'éthérification du 2-octanol (Tableau IV) ont été réalisés, dans lesquels on a remplacé la potasse par de la soude.

Conformément à ces essais, on a fait réagir, sous agitation pendant 5 heures à 25°C, 0,077 mole de 2-octanol (130,23 g par mole), 0,115 mole de soude pulvérulente, 0,154 mole de bromure de butyle et une quantité de polyéther de 5 % par rapport à la masse d'alcool faisant office de catalyseur. Après filtration et analyse chromatographique en phase vapeur, on a déterminé le rendement de l'éthérification.

Les résultats obtenus sont rassemblés dans le tableau VII ci-dessous :

TABLEAU VII

| Polyéther | Nombre moyen d'atomes d'oxygène par molécule | Rendement % |
|---|---|---|
| Sans | 0 | 2 |
| Triéthylèneglycol diméthyléther | 4 | 7 |
| Tétraéthylèneglycol diméthyléther | 5 | 12 |
| Polyéthylèneglycol 200 | 5 | 4 |
| Polyéthylèneglycol 400 | 10 | 13 |
| Polyéthylèneglycol 600 | 14 | 33 |

Ces résultats prouvent que les rendements obtenus avec la soude sont nettement inférieurs à ceux obtenus avec la potasse, ce qui pourrait s'expliquer par la présence d'eau dans le réseau de cette dernière et par un caractère basique moins marqué de la soude comparé à celui de la potasse.

De la même façon, il convient de préciser que l'hydroxyde de baryum constitue une entité basique moins bien adaptée à ce type de réaction puisque son utilisation lors de l'éthérification effectuée à titre comparatif du polyéthylèneglycol 400 a conduit au produit attendu avec un rendement de 56 % alors que dans des conditions opératoires similaires, la transformation du PEG 400 en éther correspondant s'est révélée quantitative en présence de l'hydroxyde de potassium (Tableau V).

On a également pu vérifier que, pour des raisons faciles à concevoir (augmentation des surfaces de contact, activation plus facile de l'ion hydroxyde) on a tout intérêt à utiliser la potasse sous forme finement divisée et non sous forme d'écailles ou de pastilles.

Selon une autre caractéristique de l'invention, on a constaté que l'on a avantage à mettre en oeuvre un excès de potasse de préférence de l'ordre de 2 fois la quantité stoechiométrique, dans le cas de l'éthérification d'un alcool primaire et d'environ 3 fois la quantité stoechiométrique pour l'éthérification d'une fonction alcool secondaire, alors que l'halogénure peut être mis en oeuvre en quantité stoechiométrique par rapport à l'alcool à éthérifier.

Pour vérifier ce résultat, on a fait, à titre comparatif, réagir à une température de 100°C et pendant 3 heures des quantités stoechiométriques de polypropylèneglycol 1000 et de bromure de butyle ainsi que des quantités variables de potasse en poudre, et on a déterminé le rendement de l'éthérification en fonction de la quantité de potasse mise en oeuvre.

Les résultats obtenus sont rassemblés dans le tableau VIII suivant :

TABLEAU VIII

| Quantité de potasse | Rendement % |
|---|---|
| 1 fois stoechiométrie | 45 |
| 2 fois stoechiométrie | 71 |
| 3,5 fois stoechiométrie | 79 |

Pour "affiner" ce résultat, on a pensé à reprendre cet essai dans des conditions identiques, avec une quantité de potasse égale à 3,5 fois la quantité stoechiométrique, mais en portant le milieu réactionnel non pas à 100°C mais à 20°C, et on a ainsi pu constater que l'on pouvait obtenir un rendement d'éthérification de 93 %, soit une augmentation supérieure à 10 % ; au cours de cette réaction, on a pu observer que la réaction d'éthérification étant très exothermique, la chaleur de réaction amenait le milieu réactionnel à une température de l'ordre 45 à 50°C pendant la première heure de réaction.

Conformément à l'invention, on a ensuite cherché à déterminer si le choix de la chaîne alkylée du réactif halogéné avait une influence sur le rendement de la réaction.

Dans ce but, on a fait réagir, à titre comparatif, pendant 3 heures et à 20°C, des quantités stoechiométriques de polypropylèneglycol 1000 hydroxylé et de différents bromure d'alkyles et une quantité 3,5 fois stoechiométrique de potasse et l'on a déterminé les rendements d'éthérification obtenus en fonction de la nature du réactif halogéné mis en oeuvre.

Les résultats obtenus sont rassemblés dans le tableau IX ci-dessous :

TABLEAU IX

| Réactif halogéné | Rendement % |
|---|---|
| $BrC_2H_5$ | 94 |
| $BrC_3H_7$ | 79 |
| $BrC_4H_9$ | 93 |
| $BrC(CH_3)_3$ | 8 |
| $BrCH_2C_6H_5$ | 89 |
| $Br(CH_2)_5CH_3$ | 77 |
| $Br(CH_2)_7CH_3$ | 80 |
| $BrCH_2CH{=}CH_2$ | 89 |

Ce tableau montre que, quel que soit le réactif, on observe des résultats très satisfaisants (rendement de 80 à 95 %) sauf dans le cas de réactifs très encombrés tels $BrC(CH_3)_3$.

Le choix de la chaîne alkylée de l'halogénure mis en oeuvre n'a, par suite, qu'une influence limitée sur le déroulement de la réaction, ce qui constitue un autre avantage essentiel de l'invention par rapport à l'art antérieur conformément auquel on ne pouvait mettre en oeuvre que des halogénures à chaîne courte ou autres électrophiles réactifs.

Un autre paramètre essentiel de l'invention correspond au choix de l'halogénure.

Or, on a pu constater, conformément à une autre caractéristique de l'invention que, lorsque la réaction d'éthérification est mise en oeuvre à température ambiante, les bromures d'alkyles permettent d'obtenir des rendements nettement supérieurs à ceux obtenus avec les chlorures d'alkyles ou les iodures d'alkyles correspondants.

Ce résultat a pu être confirmé par des essais comparatifs relatifs à l'éthérification d'un polypropylèneglycol 1000 hydroxylé par lequel on a fait réagir, à 20°C, des quantités stoechiométriques d'alcool et d'halogénure de butyle et une quantité 3,5 fois stoechiométrique de potasse.

Les rendements obtenus dans le cas du bromure, du chlorure et de l'iodure sont rassemblés dans le tableau X suivant :

TABLEAU X

| Halogène | Durée de la réaction (h) | Rendement % |
|---|---|---|
| Brome | 3 | 93 |
| Iode | 8 | 63 |
| Chlore | 8 | 60 |

Ce résultat est consécutif à la plus grande fragilité de la liaison C-Br par rapport à la liaison C-Cl.

On a pu vérifier que si l'on augmente la température, les rendements obtenus avec les chlorures croissent fortement mais restent inférieurs à ceux obtenus avec les bromures à température ambiante alors qu'au contraire, les rendements obtenus avec les bromures décroissent.

Ce résultat a pu être confirmé par des essais comparatifs relatifs à l'éthérification d'un polypropylèneglycol 1000 par lequel on fait réagir à diverses températures des quantités stoechiométriques d'halogénure d'octyle et de potasse.

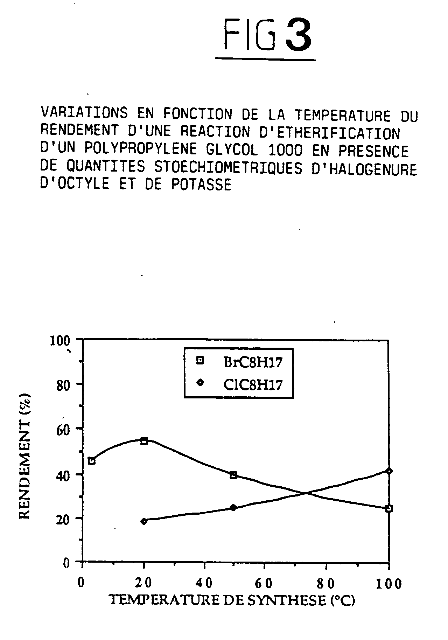

Ces essais sont rapportés sur les courbes représentées en figure 3.

Or, compte tenu du fait que les chlorures sont nettement moins onéreux que les bromures correspondants, on peut, dans certains cas, avoir intérêt à chauffer le milieu réactionnel.

Le phénomène susmentionné peut trouver son explication dans le fait que le chauffage permet de fragiliser la liaison carbone-chlore, donc de faciliter la substitution correspondant à la seconde phase de la réaction de Williamson, alors qu'un chauffage similaire favorise, dans le cas des bromures d'alkyles, le développement de réactions secondaires parasites de type nucléophile de substitution de l'ion hydroxyde sur le dérivé halogéné du type :

$$KOH + R'Br \rightarrow KX + HOR'$$

$$\text{et donc } R'OH + R'Br \rightarrow R'OR' + H_2O$$

On a pu, à cet effet, établir que, conformément à l'invention, la base activée par la solvatation du cation potassium par les polyéthers joue un rôle plus basique que nucléophile à 20°C alors que, lors d'un apport thermique, le caractère nucléophile de l'ion hydroxyde augmente. Dans ces conditions, le pourcentage de réaction d'éthérification diminue alors que le pourcentage de l'hydrolyse du dérivé halogéné augmente.

Dans tous les cas, les rendements obtenus avec les bromures à température ambiante restent nettement supérieurs à ceux obtenus avec les chlorures à températures élevées. Cette observation montre probablement que les chlorures d'alkyle subissent, eux aussi, l'hydrolyse par l'ion hydroxyde de la base lors d'un apport thermique.

L'utilisation de bromures d'alkyle et une température de synthèse proche de 20°C sont donc les conditions uniques d'obtention des éthers avec une sélectivité de 100 %.

## Revendications

1. Procédé d'éthérification sélective d'un mono ou d'un polyalcool primaire ou secondaire quelconque polyoxygéné ou non par la mise en oeuvre d'une réaction de type Williamson par laquelle, dans une première phase, on transforme en présence d'une base forte l'alcool ROH en alcoolate RO- et dans une seconde phase, on transforme l'alcoolate RO- en un éther ROR' par substitution nucléophile sur un dérivé halogéné R'X,
   caractérisé en ce que
   l'on fait réagir, en une seule étape à pression atmosphérique et sous agitation, la base forte sous forme solide, de préférence finement divisée, l'alcool à éthérifier et l'halogénure dans un milieu légèrement hydraté exempt de solvant et en présence d'une faible quantité d'un polyéther faisant office de catalyseur, puis, on isole l'éther formé de façon connue en elle-même, étant entendu que ni l'alcool de départ ROH, ni l'éther recherché sont le polyéther faisant office de catalyseur.

2. Procédé selon la revendication 1,
   caractérisé en ce que
   le polyéther utilisé comme catalyseur comporte au moins 5 groupements éthoxy consécutifs et donc au moins 6 atomes d'oxygène par molécule.

3. Procédé selon l'une quelconque des revendications 1 et 2,
   caractérisé en ce que
   l'on ajoute le polyéther faisant office de catalyseur de préférence en quantité supérieure ou égale à 5 % par rapport à la masse de l'alcool à éthérifier.

4. Procédé selon l'une quelconque des revendications 1 à 3,
   caractérisé en ce que
   si l'alcool à éthérifier est un polyéther hydroxylé, il fait lui même office de catalyseur.

5. Procédé selon l'une quelconque des revendications 1 à 4,
   caractérisé en ce que le polyéther hydroxylé à éthérifier est un polyoxyalkylène ramifié ou non.

6. Procédé selon l'une quelconque des revendications 1 à 5,
   caractérisé en ce que la base forte est un hydroxyde alcalin ou alcalino-terreux.

7. Procédé selon la revendication 6,
   caractérisé en ce que
   la base forte est la potasse, celle-ci étant introduite en quantité d'environ 2 fois la quantité stoechiométrique lors de l'éthérification d'un alcool primaire et d'environ 3 fois la quantité stoechiométrique lors de l'éthérification d'un alcool secondaire.

8. Procédé selon l'une quelconque des revendications 1 à 7,
   caractérisé en ce que
   le dérivé halogéné est un dérivé bromé.

9. Procédé selon la revendication 8,
   caractérisé en ce que

la réaction d'éthérification est mise en oeuvre à température ambiante.

10. Procédé selon l'une quelconque des revendications 6 à 9,
caractérisé en ce que
l'on choisit le taux d'hydratation du milieu réactionnel de façon que le rapport du nombre de moles d'eau au nombre de moles d'hydroxyde alcalin soit compris entre 0,1 et 5.

**Claims**

1. Process for the selective etherification of any primary or secondary mono- or polyalcohol, polyoxygenated or not, by the implementation of a Williamson type reaction by which, in a first phase, the ROH alcohol is transformed in the presence of a strong base into an RO- alcoholate and, in a second phase, the RO-alcoholate is transformed into a ROR' ether by nucleophilic substitution on an R'X halogen derivative,
characterized in that
in a single step, at atmospheric pressure and while agitating, the strong base in solid form, preferably finely divided, the alcohol to be etherified and the halide are reacted in a slightly hydrated medium devoid of solvent and in the presence of a small quantity of a polyether acting as a catalyst, then, the ether formed is isolated in a manner which is known per se, it being understood that neither the starting ROH alcohol nor the desired ether is the polyether acting as a catalyst.

2. Process according to Claim 1,
characterized in that
the polyether used as a catalyst has at least 5 consecutive ethoxy groups and therefore at least 6 oxygen atoms per molecule.

3. Process according to either of Claims 1 and 2,
characterized in that
the polyether acting as a catalyst is preferably added in an amount greater than or equal to 5% in relation to the mass of the alcohol to be etherified.

4. Process according to any one of Claims 1 to 3,
characterized in that
if the alcohol to be etherified is a hydroxylated polyether, it acts itself as a catalyst.

5. Process according to any one of Claims 1 to 4,
characterized in that the hydroxylated polyether to be etherified is a polyoxyalkylene, branched or not.

6. Process according to any one of Claims 1 to 5,
characterized in that the strong base is an alkaline or alkaline-earth hydroxide.

7. Process according to Claim 6,
characterized in that
the strong base is potassium, the latter being introduced in an amount approximately twice the stoichiometric amount when etherifying a primary alcohol, and approximately 3 times the stoichiometric amount when etherifying a secondary alcohol.

8. Process according to any one of Claims 1 to 7,
characterized in that
the halogenated derivative is a brominated derivative.

9. Process according to Claim 8,
characterized in that
the etherification reaction is carried out at ambient temperature.

10. Process according to any one of Claims 6 to 9,
characterized in that
the hydration rate of the reaction medium is selected such that the ratio of the number of mols of water to the number of mols of alkaline hydroxide is between 0.1 and 5.

## Patentansprüche

1. Verfahren zur selektiven Verätherung eines beliebigen polyoxidierten oder nicht polyoxidierten, primären oder sekundären Mono- oder Polyalkohols durch Ausführung einer Williamson-Reaktion, durch die in einem ersten Schritt in Anwesenheit einer starken Base der Alkohol ROH in Alkoholat RO- umgewandelt wird und in einem zweiten Schritt das Alkoholat RO- durch nukleophile Substitution an einem halogenierten Derivat R'X in einen Äther ROR', dadurch gekennzeichnet, daß man in einem einzigen Schritt bei Atmosphärendruck und unter Schütteln die starke Base in fester Form, vorzugsweise fein verteilt, den zu veräthernden Alkohol und das Halogenid in einer geringfügig wasserhaltigen Umgebung ohne Lösungsmittel und in Anwesenheit einer geringen Menge eines Polyäthers, der als Katalysator dient, reagieren läßt und dann der gebildete Äther in an sich bekannter Weise abgetrennt wird, wobei weder der Ausgangsalkohol ROH, noch der gesuchte Äther der Polyäther sind, der als Katalysator dient.

2. Verfahren nach Patentanspruch 1, dadurch gekennzeichnet, daß der als Katalysator verwendete Polyäther mindestens 5 aufeinanderfolgende Ethoxy-Gruppen aufweist und also mindestens 6 Sauerstoffatome pro Molekül.

3. Verfahren nach irgendeinem der Patentansprüche 1 und 2, dadurch gekennzeichnet, daß der als Katalysator dienende Polyäther vorzugsweise in einer Menge größer oder gleich 5% der Masse des zu veräthernden Alkohols zugesetzt wird.

4. Verfahren nach irgendeinem der Patentansprüche 1 bis 3, dadurch gekennzeichnet, daß, wenn der zu veräthernde Alkohol ein hydroxylierter Polyäther ist, er selbst als Katalysator dient.

5. Verfahren nach irgendeinem der Patentansprüche 1 bis 4, dadurch gekennzeichnet, daß der zu veräthernde hydroxylierte Polyäther ein verzweigtes oder unverzweigtes Polyoxyalkylen ist.

6. Verfahren nach irgendeinem der Patentansprüche 1 bis 5, dadurch gekennzeichnet, daß die starke Base ein alkalisches oder erdalkalisches Hydroxid ist.

7. Verfahren nach Patentanspruch 6, dadurch gekennzeichnet, daß die starke Base Kali ist, die bei Verätherung eines primären Alkohols in einer Menge von ungefähr dem 2-fachen der stöchiometrischen Menge und bei Verätherung eines sekundären Alkohols von ungefähr dem 3-fachen der stöchiometrischen Menge zugesetz wird.

8. Verfahren nach irgendeinem der Patentansprüche 1 bis 7, dadurch gekennzeichnet, daß das halogenierte Derivat ein bromiertes Derivat ist.

9. Verfahren nach Patentanspruch 8, dadurch gekennzeichnet, daß die Verätherungsreaktion bei Umgebungstemperatur ausgeführt wird.

10. Verfahren nach irgendeinem der Patentansprüche 6 bis 9, dadurch gekennzeichnet, daß der Wassergehalt des Reaktionsmilieus derart gewählt wird, daß das Verhältnis der Zahl der Mole Wasser zur Zahl der Mole alkalischen Hydroxids zwischen 0,1 und 5 liegt.

FIG.1

ETUDE DE L'AVANCEMENT DE LA REACTION
D'ETHERIFICATION DU 1-DECANOL A L'AIDE
DE KOH 1.5 X/Br C 4 H 9.2 X ET DIVERSES
QUANTITES DE CATALYSEUR

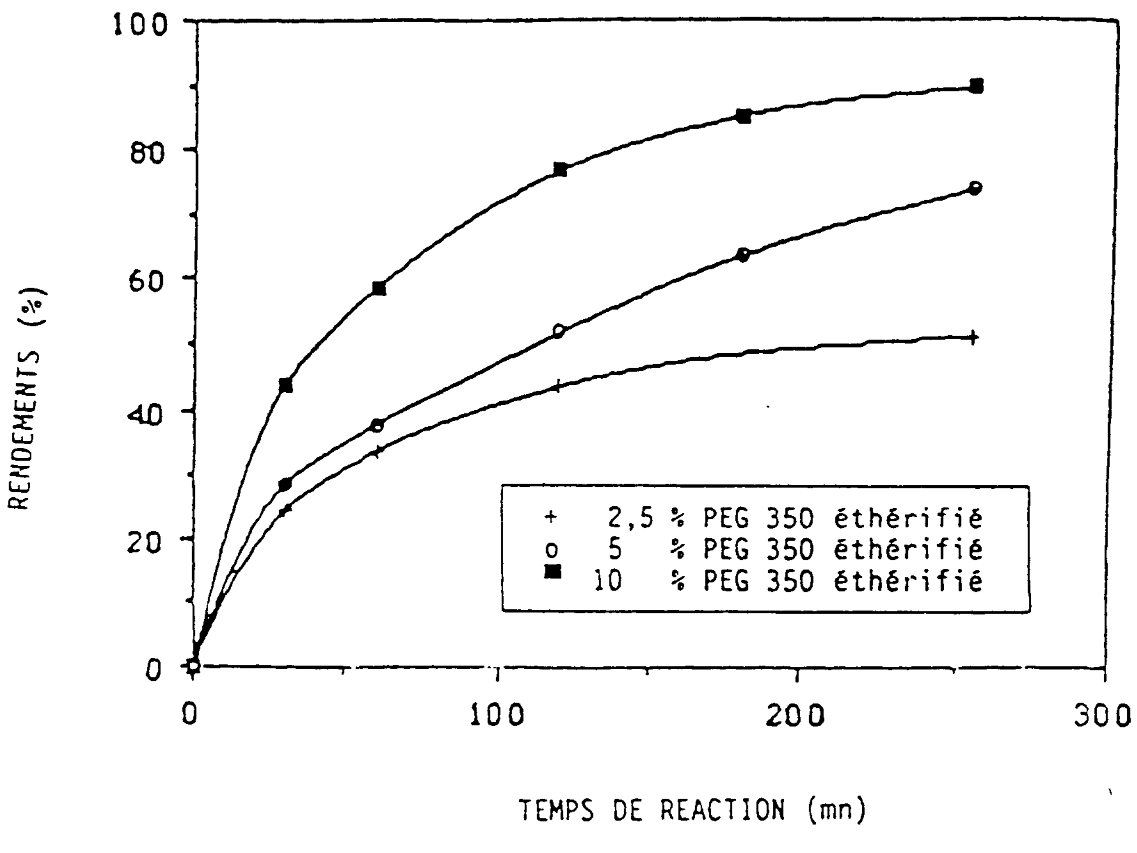

FIG.2

VARIATION DU RENDEMENT EN FONCTION DU TAUX D'HYDRATATION ET DE L'AJOUT DE CATALYSEUR

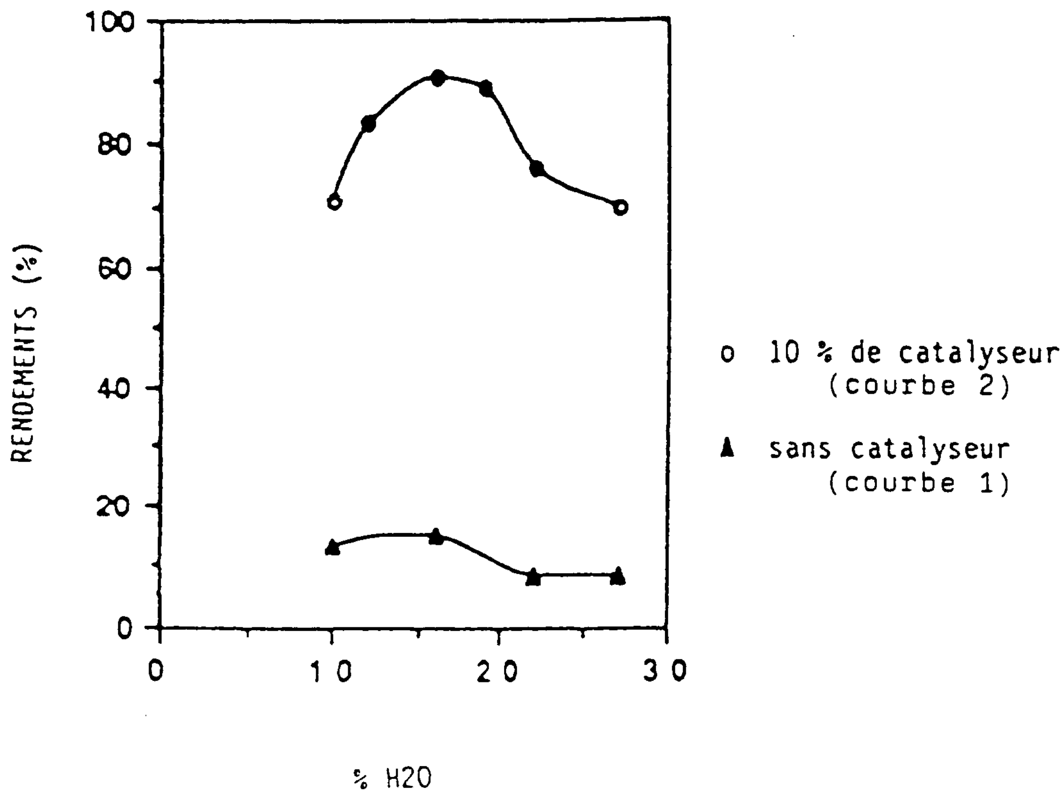

# FIG 3

VARIATIONS EN FONCTION DE LA TEMPERATURE DU RENDEMENT D'UNE REACTION D'ETHERIFICATION D'UN POLYPROPYLENE GLYCOL 1000 EN PRESENCE DE QUANTITES STOECHIOMETRIQUES D'HALOGENURE D'OCTYLE ET DE POTASSE